# EUROPEAN PATENT SPECIFICATION

(11) **EP 2 113 254 B1**
(45) Date of publication and mention of the grant of the patent: **15.08.2012**
(21) Application number: 08305134.2
(22) Date of filing: 28.04.2008
(51) Int. Cl.: A61K 35/12, A61K 38/16, A61K 38/21, A61K 45/06, A61K 39/00, C12N 5/0784

(54) **Compositions for treating an inflammatory auto-immune condition**
Zusammensetzungen zur Behandlung einer entzündlichen Autoimmunerkrankung
Compositions pour le traitement d'un état inflammatoire auto-immun

(43) Date of publication of application: 04.11.2009
(73) Proprietor: TXCell, 06560 Valbonne (FR)
(72) Inventor: Foussat, Arnaud, 06410 Biot (FR); Brun, Valérie, 06410 Biot (FR)

(56) References cited:
- ZANIN-ZHOROV A ET AL: "Heat shock protein 60 enhances CD4<+>CD25<+> regulatory T cell function via innate TLR2 signaling" JOURNAL OF CLINICAL INVESTIGATION 20060703 US, vol. 116, no. 7, 3 July 2006 (2006-07-03), pages 2022-2032, XP002486027 ISSN: 0021-9738 1558-8238
- WILCZYNSKI JACEK R ET AL: "The characterization and role of regulatory T cells in immune reactions." FRONTIERS IN BIOSCIENCE : A JOURNAL AND VIRTUAL LIBRARY 2008, vol. 13, 1 January 2008 (2008-01-01), pages 2266-2274, XP002486028 ISSN: 1093-4715
- WU KUI ET AL: "IL-10-producing type 1 regulatory T cells and allergy." CELLULAR & MOLECULAR IMMUNOLOGY AUG 2007, vol. 4, no. 4, August 2007 (2007-08), pages 269-275, XP002486029 ISSN: 1672-7681
- WIETEN L ET AL: "Cell stress induced HSP are targets of regulatory T cells: A role for HSP inducing compounds as anti-inflammatory immuno-modulators?" FEBS LETTERS - CELLULAR STRESS 20070731 NL, vol. 581, no. 19, 31 July 2007 (2007-07-31), pages 3716-3722, XP002487127 ISSN: 0014-5793
- VAN EDEN W ET AL: "Stress, heat shock proteins, and autoimmunity: How immune responses to heat shock proteins are to be used for the control of chronic inflammatory diseases" ANNALS OF THE NEW YORK ACADEMY OF SCIENCES - STRESS RESPONSES 200710 US, vol. 1113, October 2007 (2007-10), pages 217-237, XP009102173 ISSN: 0077-8923 1749-6632 1-57331-67

## Description

### FIELD OF THE INVENTION

The present invention relates to the field of treatment of an inflammatory autoimmune condition. The present invention relates in particular to methods for treating an inflammatory autoimmune condition and immune related diseases, using a medicament comprising human Trl cells directed against human heat shock protein.

### BACKGROUND

Inflammatory autoimmune diseases affect millions of people worldwide and can have devastating effects on lifespan and quality of life.

Conventional therapies for treating inflammatory autoimmune diseases in humans are based on non-specific immunosuppression, such as that mediated by steroids or cytotoxic agents. More recent stategies include the blockade of several pro-inflammatory cytokine pathways, such as those that involve TNF and IL-1. However, none of these strategies reverses the pro-inflammatory process or cures the disease. When treatment is stopped, a reactivation of inflammation occurs within months, thereby prompting life-long treatment with immunosuppressive agents. This inevitably leads to serious side effects.

Therefore, there is a need for novel treatments for inflammatory autoimmune diseases.

Various studies have demonstrated that heat shock proteins (HSPs), and in particular HSP60 and HSP70, constitute a group of autoantigen involved in the regulation of inflammatory autoimmune diseases.

Therapies targeting HSPs for treating inflammatory autoimmune diseases have therefore been proposed such as immunisation with E. coli lysate or peptides derived from HSP60 or use of DNA vaccines comprising a nucleic acid sequence encoding HSP60, HSP70 or HSP90 or fragment thereof.

In the present invention, the Applicant aims to provide an alternative treatment for an inflammatory autoimmune condition based on the use of Trl cells directed to a human HSP.

Zanin-Zhorov et al. J. Clin Invest. 116 : 2022-2032 (2006) describes that heat shock protein 60 enhances cd4⁺ cd25⁺ regulatory T cell function via innate TLh2 signaling.

### SUMMARY OF THE INVENTION

An object of the invention is a medicament comprising at least one human Tr1 cell population directed against a human HSP. Another object of the invention is a pharmaceutical composition comprising at least one human Tr1 cell population directed against a human HSP in combination with one or more pharmaceutically acceptable carrier.

Another object of the invention is the use of a composition comprising at least one human Tr1 cell population directed against a human HSP for the preparation of a medicament or a pharmaceutical composition for treating an inflammatory autoimmune condition.

According to an embodiment of the invention, said inflammatory autoimmune condition is an arthritis condition, preferably rheumatoid arthritis, psoriatic arthritis, ankylosing spondylitis and juvenile idiopathic arthritis. According to another embodiment, said inflammatory autoimmune is a multiple sclerosis condition. According to another embodiment, said inflammatory autoimmune condition is an intestinal inflammatory condition, preferably Crohn's disease and ulcerative colitis. According to another embodiment, said inflammatory autoimmune condition is a vasculitis, preferably Wegener's disease and Atherosclerosis. According to another embodiment, said inflammatory autoimmune condition is Asthma. According to another embodiment, said inflammatory autoimmune condition is transplant rejection or Graft versus Host disease. According to another embodiment, said inflammatory autoimmune condition is an inflammatory condition of the biliary duct, preferably Primary Biliary Cirrhosis and

Primary sclerosing cholangitis.

According to another embodiment, the administration to said subject of an effective amount of the medicament or the pharmaceutical composition of the invention is in combination with one or more therapeutic agent used for treating an inflammatory autoimmune condition.

According to another embodiment, the administration to said subject of an effective amount of the medicament or the pharmaceutical composition is in combination with one or more medicament or pharmaceutical composition comprising at least one Tr1 cell population or clone directed against an antigen known to be involved in the inflammatory autoimmune condition.

Another object of the invention is a process for treating an inflammatory autoimmune condition in a subject in need thereof, said process comprising the steps of:
- obtaining Tr1 cells directed to a human HSP, said Tr1 cells being obtained from a blood sample of said subject,
- cloning said Tr1 cells directed to a human HSP,
- further expanding Tr1 clones obtained at the previous step,
- injecting Tr1 clones thus obtained in said subject, preferably by intravenous route.

### BRIEF DESCRIPTION OF THE DRAWINGS

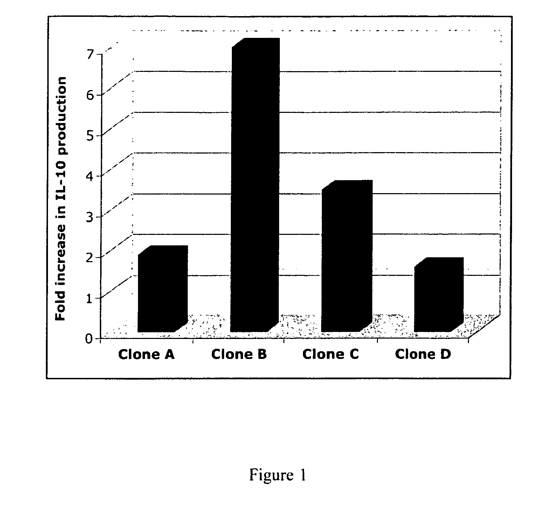

**Figure 1**: specific IL-10 production of Tr1 clones after HSP60 activation
Figure 1 describes the specific increase of the IL-10 production by Tr1 cell clones in the presence of the specific antigen HSP60. Clones were activated with or without HSP60 in the presence of irradiated autologous antigen presenting cells. After 48 hours, the IL-10 production was measured by ELISA.

### Figure 2: Cytokine secretion profile of HSP60 specific Tr1 clones

IL-10, IL-4 and IFNγ secretion of HSP60 specific Tr1 cell clones were measured in 48 hours supernatant of anti-CD3 + anti-CD28 monoclonal antibodies activated cells.

### Figure 3: In vitro suppressive activity of HSP60 Tr1 clones

The suppressive activity of HSP60 Tr1 clones was assessed by evaluating the inhibitory action of their supernatants on autologous CD4+ T lymphocyte proliferation. CD4+ T lymphocyte were activated using anti-CD3 + anti-CD28 monoclonal antibodies and

Interleukin-2 in the presence or absence of the supernatants of activated Tr1 clones. Cell proliferation of the autologous CD4+ T cells was measured after five days. Results show that the addition of Tr1 cell's supernatants to CD4+ T lymphocytes inhibits T-cell proliferation.

### DETAILED DESCRIPTION OF THE INVENTION

### Definition

The term "Tr1 cells" as used herein refers to cells having the following phenotype at rest CD4+CD25-FoxP3- and capable of secreting high levels of IL-10 and significant levels TGF-β upon, activation. Tr1 cells are characterized, in part, by their unique cytokine profile: they produce high levels of IL-10, significant levels of TGF-β and intermediate levels of IFN-γ, but little or no IL-4 or IL-2. The cytokine production is typically evaluated in cultures of cells after activation with polyclonal activators of T lymphocytes such as anti-CD3+ anti-CD28 antibodies or Interleukin-2, PMA + ionomycin, Alternatively, the cytokine production is evaluated in cultures of cells after activation with the specific T-cell antigen presented by antigen presenting cells. High levels of IL-10 correspond to at least about 500 pg/ml, typically greater than about 1, 2, 4, 6, 8, 10, 12, 14, 16, 18, or 20 thousand pg/ml or more. Significant levels of TGF-β correspond to at least about 100 pg/ml, typically greater than about 200, 300, 400, 600, 800, or 1000 pg/ml or more.

Intermediate levels of IFN-γ correspond to concentrations comprised between 0 pg/ml and at least 400 pg/ml, typically greater than about 600, 800, 1000, 1200, 1400, 1600, 1800, or 2000 pg/ml or more. Little or no IL-4 or IL-2 corresponds to less than about 500 pg/ml, preferably less than about 250, 100, 75, or 50 pg/ml, or less.

The term "antigen" as used herein refers to a protein, or peptide for which the cells of this invention are being used to modulate, or for use in any of the methods of this invention. In one embodiment, the term "antigen" may refer to a synthetically derived molecule, or a naturally derived molecule, which shares sequence homology with an antigen of interest, or structural homology with an antigen of interest, or a combination thereof. In one embodiment, the antigen may be a mimetope. A "fragment" of the antigen refers to any subset of the antigen, as a shorter peptide. A "variant" of the antigen refers to a molecule substantially similar to either the entire antigen or a fragment thereof. Variant antigens may be conveniently prepared by direct chemical synthesis of the variant peptide, using methods well-known in the art.

The term "subject" as used herein refers to a human being.

The term "effective amount" as used herein refers to an amount sufficient to cause a beneficial or desired clinical result (e.g. improvement in clinical condition).

The term "clone" or "clone population" as used herein refers to a population of differentiated cells being derived from a unique differentiated cell.

The term "treatment" or "treating" as used herein generally refers to a clinical intervention in an attempt to alter the natural course of the individual being treated, and may be performed during the course of clinical pathology. Desirable effects include, but are not limited to, alleviating symptoms, suppressing, diminishing or inhibiting any direct or indirect pathological consequences of the disease, lowering the rate of disease progression, ameliorating or palliating the disease state, and causing remission or improved prognosis. In the context of the invention, it refers to any improvement in the clinical symptoms of the autoimmune condition, as well as any improvement in the well being of the patients.

The term "autoimmune disease" as used herein refers to an immune response directed against an autoantigen.

### The present invention

The present invention relates to a method for treating an inflammatory autoimmune condition in a subject in need thereof, comprising the administration to said subject of a composition comprising at least one human Tr1 cell population directed against a human HSP.

Another object of the present invention is to provide a medicament comprising at least one human Tr1 cell population directed against a human HSP.

The present invention also intends to provide a pharmaceutical composition comprising at least one human Tr1 cell population directed against a human HSP in combination with one or more pharmaceutically acceptable carrier.

According to the invention, the "human Tr1 cell population" corresponds to Tr1 cells as described here above in the definitions and does not include CD4+CD25+ regulatory T cells or FoxP3+ regulatory T cells (natural or conventional Treg), TGF-β secreting Th3 cells, or regulatory NKT cells.

According to the invention, the term "human HSP" refers to human HSP10, HSP40, HSP60, HSP70, HSP90 and HSP100 families, fragments and mixtures thereof.

In a preferred embodiment of the invention, said Tr1 cells are directed against HSP60, HSP70, HSP90, fragments and mixtures thereof.

In a more preferred embodiment of the invention, said Tr1 cells are directed against HSP60 and fragments thereof.

Without whishing to be bound to a theory, the Applicant assume that the injected Tr1 cell population directed against a human HSP would be activated in vivo by the HSPs whose expression is upregulated in an inflammatory autoimmune condition.

In one embodiment of the invention, human Tr1 cells may be obtained by
a) isolating a progenitor cell population from a subject,
b) obtaining a population of dendritic cells by culturing said progenitor cell population in the presence of IL-10,
c) contacting cells of step b) with a CD4+ T lymphocyte population isolated from said subject in the presence of a human HSP to allow differentiation of CD4+ T cells directed to said antigen into the Tr1 cell population, and
d) recovering the Tr1 cell population from the step c).

In step b), IL-10 is present from 50 to 250 U/ml, preferably at 100 U/ml in the culture medium. Said method for obtaining Tr1 cells is described in Wakkach et al (Immunity 2003 May; 18(5):605-17).

Said method may also be carried out using Dexamethasone and Vitamin D3, or tolerogenised or immature DCs instead of the DCs of step b).

In another embodiment of the present invention, human Tr1 cells may be obtained by:
a) culturing a CD4+ T cell population directed against a human HSP isolated from a subject in a media with an appropriate amount of IFN-α, and
b) recovering the Tr1 cell population.

IFN-α, is preferably present in the media at 5 ng/ml. In the step a), the media may further comprise an appropriate amount of IL-10, preferably at 100 U/ml.

In step b), the Tr1 cell population is cultured in a media comprising IL-15 to allow proliferation, IL-15 being preferably at 5 ng/ml in the media. Said method for obtaining Tr1 cells is described in the patent US6746670.

In still another embodiment of the invention, human Tr1 cells may be obtained by:
a) in vitro activating a CD4+ T cell population in presence of a human HSP, presented by artificial antigen presenting cells, and
b) recovering an activated CD4+ T cells comprising at least 10% of Tr1 cells.

Preferably, the artifical antigen presenting cells express a HLA II system molecule and a human LFA-3 molecule and don't express the co-stimulation molecules B7-1, B7-2, B7-H1, CD40, CD23 and ICAM-1.

Said process, for obtaining Tr1 cells is described in the patent application WO02/092793.

In still another embodiment of the invention, human Tr1 cells may be obtained by:
a) in vitro activating a CD4+ T cell population in the presence of a human HSP and an appropriate amount of IL-10; and
b) recovering the Tr1 cell population.

Preferably, IL-10 is present in the media at 100 U/ml. Said method is described in Groux et al. (Nature 1997, 389(6652):737-42).

In still another embodiment of the invention, human Tr1 cells may be obtained by:
a) stimulating a leukocyte population or a peripheral blood mononuclear cell (PBMC) population with a human HSP,
b) recovering the human HSP-specific Tr1 cell population from the stimulated population,
c) optionally expanding said human HSP-specific Tr1 cell population.

Leukocytes encompass several types of cells, which are characterized by their importance, their distribution, their number, their lifetime and their potentiality. These types are the following : the polynuclear or granular leukocytes, among which one finds the eosinophilic, the neutrophilic and the basophilic leukocytes, and the mononuclear cells, or peripheral blood mononuclear cells (PBMCs), which are large white blood cells and consist in the cell types of the immune system (lymphocytes and monocytes). The leukocytes or the PBMCs can be separated from the peripheral blood by any method known to those skilled in the art. Advantageously, for the separation of the PBMCs, centrifugation may be used, preferably density gradient centrifugation, preferably discontinuous density gradient centrifugation. An alternative is the use of specific monoclonal antibodies. In certain embodiments PBMC are typically isolated from the whole blood product by means of Ficoll-Hypaque, using standard procedures. In other embodiments the PBMCs are recovered by means of leukapheresis.

Said method is described in the patent application WO2007/010406.

In still another embodiment, human Tr1 cells may be obtained by:
a) culturing a leukocyte population or a peripheral blood mononuclear cell (PBMC) population with mesenchymal stem cells in the presence of a human HSP,
b) recovering the Tr1 cell population.

Said method can also be carried out with naïve or memory T cells instead of PBMC or leukocytes.

The Tr1 cell population thus obtained may further be expanded by culture in presence of cytokines such as Interleukin-2 and Interleukin-4. Alternatively, Interleukin-15 and

Interleukin-13 could also be used in Tr1 cell expansion cultures.

In the methods described above, human Tr1 cells can be characterized by the identification method described in WO2005/000344. Said identification method of Tr1 cells is based on the detection of the simultaneous presence of expression products of genes coding CD4 molecule and molecules from the group comprising CD18 and/or CD11a, and CD49b. Tr1 cells can be identified and/or purified by Elisa, flow cytometry, or immunoaffinity methods with antibodies directed against said markers.

Tr1 cells can also be enriched by positive selection or negative selection using flow cytometry or magnetic beads. Such methods are also described in WO2005/000344.

In another embodiment of the present invention, the Tr1 cells directed to a joint-associated antigen may be expanded by the in vitro method described in WO2006/108882. Said method comprises:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

In a preferred embodiment of the present invention, said Tr1 cells directed to a human HSP may be cloned by using conventional methods for cloning T cells.

In a preferred embodiment of the present invention, said composition comprising at least one human Tr1 cell population directed against a human HSP or at least one clone of human Tr1 cell directed against a human HSP may be frozen to be stored.

According to a preferred embodiment, said human Tr1 cell population is a human Tr1 clone population.

The pharmaceutically acceptable carriers useful herein are conventional. Remington's Pharmaceutical Sciences 16th edition, Osol, A. Ed. (1980) describes composition and formulations suitable for pharmaceutical delivery of the composition of the present invention. In general, the nature of the carrier will depend on the mode of administration being employed. For instance, parenteral formulations usually comprise injectable fluids that include pharmaceutically and physiologically acceptable fluids such as water, physiological saline, balanced salt solutions, aqueous dextrose, sesame oil, glycerol, ethanol, combinations thereof, or the like, as vehicle. The carrier and composition can be sterile, and the formulation suits the mode of administration. In addition to biological neutral carriers, pharmaceutical compositions to be administrated can contain minor amounts of non toxic auxiliary substances, such as wetting or emulsifying agents, preservatives, and pH buffering agents and the like, for example sodium acetate or sorbitan monolaurate. The composition can be a liquid solution, suspension, emulsion.

The present invention relates to the use of at least one human Tr1 cell population directed against a human HSP for the preparation of a medicament or a pharmaceutical composition as described here above for treating an inflammatory autoimmune condition.

An inflammatory autoimmune condition includes but is not limited to: autoimmune (Hasimoto's) thyroiditis, hyperthyroidism (Graves' disease), autoimmune adrenal insufficiency (Addison's disease), autoimmune oophoritis, autoimmune orchitis, autoimmune hepatitis, autoimmune hemolytic anemia, paroxysmal cold hemoglobinuria, autoimmune thrombocytopenia, autoimmune neutropenia, pernicius anemia, pure red cell anemia, autoimmune coagulopathies, myasthenia gravis, autoimmune polyneuritis, multiple sclerosis, pemphigus and other bullous diseases, rheumatic carditis, Goodpasture's syndrome, postcardiotomy syndrome, systemic lupus erythematosus, rheumatoid arthritis, Sjorgen's syndrome, polymyositis, dermatomyositis, scleroderma, inflammatory bowel diseases: Crohn's disease, ulcerative colitis; chronic obstructive pulmonary diseases, chronic inflammatory diseases, Coeliac disease, vasculitis, Wegener's disease, Churg-Strauss Syndrome, Primary biliary Cirrhosis, Primary sclerosing cholangitis, cardiovascular disease, rheumatoid arthritis, psoriatic arthritis, ankylosing spondylitis, juvenile idiopathic arthritis, polydermatomyositis, septic shock, host versus graft disease, graft versus host disease, asthma, rhinitis, psoriasis, cachexia associated with cancer, eczema, Vitiligo, Reiter's syndrome, Kawasaki's Disease, idiopathic thrombocytopenic purpura, Guillain-Barré syndrome, Antiphospholipid antibody syndrome (APS), Atherosclerosis, Narcolepsy.

In a preferred embodiment, the present invention relates to the use of at least one human Tr1 cell population directed against a human HSP for the preparation of a medicament or a pharmaceutical composition as described here above for treating an inflammatory autoimmune condition, which is not diabetes.

Preferably, said inflammatory autoimmune disease is selected in the group comprising intestinal inflammatory condition such as Crohn's disease and ulcerative colitis; arthritis condition such as rheumatoid arthritis, psoriatic arthritis, ankylosing spondylitis and juvenile idiopathic arthritis; multiple sclerosis, Wegener's disease, primary Biliary Cirrhosis, Primary sclerosing cholangitis, asthma, transplant rejection (host versus graft disease), graft versus host disease.

More preferably, said inflammatory autoimmune disease is selected in the group of rheumatoid arthritis, Crohn's disease, ulcerative colitis, asthma and transplant rejection or graft versus host disease.

In a preferred embodiment, the present invention relates to the use of a composition as described here above for the preparation of a medicament or a pharmaceutical composition for treating arthritis such as rheumatoid arthritis, psoriatic arthritis, ankylosing spondylitis and juvenile idiopathic arthritis.

In another preferred embodiment, the present invention relates to the use of a composition as described here above for the preparation of a medicament or a pharmaceutical composition for treating multiple sclerosis.

In another preferred embodiment, the present invention relates to the use of a composition as described here above for the preparation of a medicament or a pharmaceutical composition for treating intestinal inflammatory condition, preferably for treating Crohn's disease, and ulcerative colitis.

In another preferred embodiment, the present invention relates to the use of a composition as described here above for the preparation of a medicament or a pharmaceutical composition for treating vasculitis such as Wegener's disease and atherosclerosis.

In another preferred embodiment, the present invention relates to the use of a composition as described here above for the preparation of a medicament or a pharmaceutical composition for treating autoimmune inflammation of the biliary duct or the liver such as Primary biliary cirrhosis and Primary sclerosing cholangitis.

In another preferred embodiment, the present invention relates to the use of a composition as described here above for the preparation of a medicament or a pharmaceutical composition for treating transplantation related disorders such as host versus graft disease and graft versus host disease.

In another preferred embodiment, the present invention relates to the use of a composition as described here above for the preparation of a medicament or a pharmaceutical composition for treating asthma.

An object of the present invention is also a method for treating an inflammatory autoimmune condition in a subject in need thereof, comprising administering to said subject an effective amount of the medicament or pharmaceutical composition as described here above.

The composition may be formulated for parenteral, intramuscular, intravenous, intraperitoneal, injection, intranasal inhalation, lung inhalation, intradermal, intra-articular, intrathecal.

Preferably, the medicament or pharmaceutical composition of the invention may be administrated by intramuscular, intraperitoneal or intravenous injection, or by direct injection into the lymph nodes of the patient, more preferably by intravenous injection.

The amount of Tr1 cells directed to a human HSP effective in the treatment of an inflammatory autoimmune condition will depend on the nature of the inflammation, and can be determined by standard clinical techniques. The precise dose to be employed in the formulation will also depend on the route of administration, and the seriousness of the disease or disorder, and should be decided according to the judgment of the practitioner and each individual's circumstances. Effective doses can be extrapolated from dose-response curves derived from in vitro or animal model test systems.

In one embodiment of the present invention, 10⁴/kg to 10⁹/kg cells are administrated to the subject. Preferably 10⁵/kg to 10⁷/kg cells and more preferably about 10⁶/kg cells are administrated to the subject.

In one embodiment of the invention, the subject is administrated with the medicament at the time when flare-up are demonstrated by a decline in the clinical status of the subject.

In one embodiment of the invention, the subject is administrated once with the medicament or the pharmaceutical composition of the present invention.

In a second embodiment of the invention, the subject is administrated once a month with the medicament or the pharmaceutical composition of the present invention.

In a third embodiment of the invention, the subject is administrated once a quarter with the medicament or the pharmaceutical composition of the present invention.

In a fourth embodiment of the invention, the subject is administrated once to twice a year with the medicament or the pharmaceutical composition of the present invention.

In another embodiment of the present invention, the medicament or pharmaceutical composition to be administered to a subject in need thereof comprises human Tr1 cells autologous to the cells of said subject.

This means that Tr1 cells will be administrated to the subject they come from or that precursors used for the production of Tr1 cells come from the subject the Tr1 cells will be administrated to.

The present invention relates also to a process for treating an inflammatory autoimmune condition in a subject in need thereof, said process comprising the steps of:
- collecting a blood sample of said subject,
- obtaining Tr1 cells directed to a human HSP,
- cloning said Tr1 cells directed to a human HSP,
- further expanding Tr1 clones obtained at the previous step,
- injecting Tr1 clones thus obtained in said subject, preferably by intravenous route.

Preferably, cloning and expansion of Tr1 clones directed to a human HSP is carried out with the following method:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

In another embodiment of the invention, the process for treating an inflammatory autoimmune condition comprises additional steps such as combined administration of one or more therapeutic agents such as described here after and/or combined administration of an effective amount of a medicament or pharmaceutical composition comprising at least one human Tr1 cell population directed against an antigen known to be involved in the inflammatory autoimmune condition as described here after.

In another embodiment of the present invention, the method for treating an inflammatory autoimmune condition in a subject in need thereof comprises the administration to said subject of an effective amount of the medicament or the pharmaceutical composition of the invention in combination with one or more therapeutic agent used for treating an inflammatory autoimmune condition.

The present invention relates to the use of the pharmaceutical composition or medicament of the invention for treating an inflammatory autoimmune condition, wherein the administration to said subject of an effective amount of the medicament or the pharmaceutical composition of the invention is in combination with one or more therapeutic agent used for treating an inflammatory autoimmune condition.

Examples of therapeutic agents commonly used for treating an arthritic inflammatory condition are the following:
- corticoids (prednisone),
- TNF blocking agents such as Infliximab, Adalimumab, Etanercept;
- anti-interleukins such as Anakinra, AMG108, Iguratimod, Actemra
- anti-B lymphocytes such as Rituximab, Epratuzumab;
- anti- costimulatory molecules such as Abatacept, Belimumab;
- tolerogenic agents (synthetic molecules directed to B lymphocyte surface DNA receptors) such as LJP 394 or TV-4710;
- anti-complement protein such as Eculizumab;
- Inhibitors of T cell signalling molecules such as CP690550
- Inhibitors of cell migration such as antagonist of chemokine receptors (Maraviroc, INCB3284)
- leflunomide, sulfasalazine, hydroxychloroquine, azathioprine, methotrexate, cyclosporine, minocycline, D-penicillamine,
- combination therapy thereof such as methotrexate + sulfasaline, methothrexate + hydroxychloroquine, methotrexate + azathioprine, methotrexate + infliximab, methotrexate + leflunomide, methotrexate + etanercept, cyclosporine + hydroxychloroquine, cyclosporine + methotrexate, methotrexate + sulfasalazine + hydroxychloroquine.

In a preferred embodiment of the present invention, the method for treating an arthritic inflammatory condition in a subject in need thereof comprises the administration to said subject of an effective amount of the medicament or the pharmaceutical composition of the invention in combination with one or more therapeutic agent selected in the group of corticoids, TNF blocking agents, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

The present invention relates to the use of the pharmaceutical composition or medicament of the invention for treating an inflammatory arthritic condition in a subject in need thereof, wherein the administration to said subject of an effective amount of the medicament or the pharmaceutical composition of the invention is in combination with one or more therapeutic agent selected in the group of corticoids, TNF blocking agents, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

Examples of therapeutic agents commonly used for treating multiple sclerosis are the following:
- interferons, e.g., human interferon beta-la (e.g., AVONEX® or Rebif®) and interferon beta-Ib (BETASERON™); human interferon beta substituted at position 17; Berlex/Chiron);
- glatiramer acetate (also termed Copolymer 1, Cop-1; COPAXONE™); Teva Pharmaceutical Industries, Inc.); and derivatives,
- fumarates, e.g., dimethyl fumarate (e.g., Fumaderm®);
- Rituxan® (rituximab) or another anti-CD20 antibody, e.g., one that competes with or binds an overlapping epitope with rituximab;
- mitoxantrone (NOVANTRONE®, Lederle);
- a chemotherapeutic, e.g., clabribine (LEUSTATIN®), azathioprine (IMURAN®), cyclophosphamide (CYTOXAN®), cyclosporine-A, methotrexate, 4-aminopyridine, and tizanidine;
- a corticosteroid, e.g., methylprednisolone (MEDRONE®, Pfizer), prednisone;
- an immunoglobulin, e.g., Rituxan® (rituximab); CTLA4 Ig; alemtuzumab (MabCAMPATH®) or daclizumab (an antibody that binds CD25);
- statins;
- immunoglobulin G intravenous (IgGIV),
- Nataluzimab (Tysabri) anti-integrin alpha-4 antibody,
- the oral CC chemokine receptor 1 antagonist BX471 (ZK811752),
- FTY720 (fingolimod),
- antibodies or antagonists of human cytokines or growth factors, for example, TNF, LT, IL- 1, IL-2, IL-6, IL-7, IL-8, IL- 12, IL- 15, IL- 16, IL-17, IL- 18, IL-23, EMAP-11, GM-CSF, FGF, and PDGF.
- antibodies to cell surface molecules such as CD2, CD3, CD4, CD8, CD25, CD28, CD30, CD40, CD45, CD69, CD80, CD86, CD90 or their ligands.
- FK506, rapamycin, mycophenolate mofetil, leflunomide, non-steroidal anti-inflammatory drugs (NSAIDs), for example, phosphodiesterase inhibitors, adenosine agonists, antithrombotic agents, complement inhibitors, adrenergic agents, agents that interfere with signaling by proinflammatory cytokines as described herein, IL- I[beta] converting enzyme inhibitors (e.g., Vx740), anti-P7s, PSGL, TACE inhibitors, T-cell signaling inhibitors such as kinase inhibitors, metal loproteinase inhibitors, sulfasalazine, azathloprine, 6-mercaptopurines, angiotensin converting enzyme inhibitors,
- amantadine, baclofen, papaverine, meclizine, hydroxyzine, sulfamethoxazole, ciprofloxacin, docusate, pemoline, dantrolene, desmopressin, dexamethasone, tolterodine, phenytoin, oxybutynin, bisacodyl, venlafaxine, amitriptyline, methenamine, clonazepam, isoniazid, vardenafil, nitrofurantoin, psyllium hydrophilic mucilloid, alprostadil, gabapentin, nortriptyline, paroxetine, propantheline bromide, modafinil, fluoxetine, phenazopyridine, methylprednisolone, carbamazepine, imipramine, diazepam, sildenafil, bupropion, and sertraline.

Examples of combination therapies currently used are:
- glatiramer acetate and albuterol,
- glatiramer acetate and minocycline,
- interferon-beta 1a and mycophenolate mofetil,
- BHT-3009 and atorvastatin,

In a preferred embodiment of the present invention, the method for treating multiple sclerosis in a subject in need thereof comprises the administration to said subject of an effective amount of the medicament or the pharmaceutical composition of the invention in combination with one or more therapeutic agent in the group of interferon-beta, glatiramer acetate, mitoxantrone, cyclophosphamide, methotrexate, aziathropine or natalizumab.

The present invention relates to the use of the pharmaceutical composition or medicament of the invention for treating multiple sclerosis, wherein the administration to said subject of an effective amount of the medicament or the pharmaceutical composition of the invention is in combination with one or more therapeutic agent in the group of interferon-beta, glatiramer acetate, mitoxantrone, cyclophosphamide, methotrexate, aziathropine or natalizumab.

Examples of therapeutic agents commonly used for treating an intestinal inflammatory condition are the following:
- antibodies or antagonists of human cytokines or growth factors, in particular, anti-TNF such as infliximab, adalimumab, anti-IL1, anti-IL-6, anti- IL-12, anti-IL-17 and anti-IL-23; IL-1 receptor antagonist analogs (anakinra), and TNF blocking agents such as etanercept,
- antibodies to cell surface molecules such as anti-α4 integrin (natalizumab), CD2, CD3 (visiluzumab), anti-CCR9, anti-LFA1 and anti-ICAM1;
- 5 aminosalicyclic acid and analogs such as mesalazine, sulfazaline, olsalazine, balsalazide;
- corticoids such as prednisone, budesonide, hydrocortisone, prednisolone, methylprednisolone, betamethasone, bedomethasone, tixocortol;
- probiotics such as saccharomyces boulardii;
- methotrexate, thalidomide, leflunomide, and analogs of purines such as Azathioprine and 6-mercaptopurine.

In a preferred embodiment of the present invention, the method for treating an intestinal inflammatory condition in a subject in need thereof comprises the administration to said subject of an effective amount of the medicament or the pharmaceutical composition of the invention in combination with one or more therapeutic agent selected in the group of anti-TNF or TNF blocking agents, natalizumab, anti-ILl, anti-IL-6, anti- IL-12, anti-IL-17 and anti-IL-23; IL-1 receptor antagonist analogs (anakinra); 5 aminosalicyclic acid and analogs such as mesalazine, sulfazaline, olsalazine, balsalazide; corticoids such as prednisone, budesonide, hydrocortisone, prednisolone, methylprednisolone, betamethasone, bedomethasone, tixocortol.

The present invention relates to the use of the pharmaceutical composition or medicament of the invention for treating an intestinal inflammatory condition in a subject in need thereof, wherein the administration to said subject of an effective amount of the medicament or the pharmaceutical composition of the invention is in combination with one or more therapeutic agent selected in the group of anti-TNF or TNF blocking agents, natalizumab, anti-IL1, anti-IL-6, anti- IL-12, anti-IL-17 and anti-IL-23; IL-1 receptor antagonist analogs (anakinra); 5 aminosalicyclic acid and analogs such as mesalazine, sulfazaline, olsalazine, balsalazide; corticoids such as prednisone, budesonide, hydrocortisone, prednisolone, methylprednisolone, betamethasone, bedomethasone, tixocortol.

Therapeutic agents commonly used for treating vasculitis such as atherosclerosis and Wegener'disease are statins, aspirin, blood coagulants such as heparin, coumadin for atherosclerosis and corticoids, aziathioprine, methothrexate, cyclophosphamide, anti-B lymphocytes antibodies (Rituximab), anti-TNF antibodies (Etanercept, Remicade) and anti-thymocyte globulin for Wegener's disease.

In a preferred embodiment of the present invention, the method for treating vasculitis such as atherosclerosis and Wegener's disease in a subject in need thereof comprises the administration to said subject of an effective amount of the medicament or the pharmaceutical composition of the invention in combination with statins, aspirin, blood coagulants such as heparin, coumadin for atherosclerosis and corticoids, aziathioprine, methothrexate, cyclophosphamide, anti-B lymphocytes antibodies (Rituximab) TNF blocking agents (Etanercept, Remicade) and anti-thymocyte globulin for Wegener's disease.

The present invention relates to the use of the pharmaceutical composition or medicament of the invention for treating vasculitis such as atherosclerosis and Wegener's disease in a subject in need thereof, wherein the administration to said subject of an effective amount of the medicament or the pharmaceutical composition of the invention is in combination with statins, aspirin, blood coagulants such as heparin, coumadin for atherosclerosis and corticoids, aziathioprine, methothrexate, cyclophosphamide, anti-B lymphocytes antibodies (Rituximab) and TNF blocking agents (Etanercept, Remicade) and anti-thymocyte globulin for Wegener's disease.

Therapeutic agents commonly used for treating autoimmune inflammation of the biliary duct or the liver such as Primary biliary cirrhosis and Primary sclerosing cholangitis is Ursodeoxycholic acid (Ursodiol).

In a preferred embodiment of the present invention, the method for treating autoimmune inflammation of the biliary duct or the liver such as Primary biliary cirrhosis and Primary sclerosing cholangitis in a subject in need thereof comprises the administration to said subject of an effective amount of the medicament or the pharmaceutical composition of the invention in combination with Ursodeoxycholic acid (Ursodiol).

The present invention relates to the use of the pharmaceutical composition or medicament of the invention for treating autoimmune inflammation of the biliary duct or the liver such as Primary biliary cirrhosis and Primary sclerosing cholangitis in a subject in need thereof, wherein the administration to said subject of an effective amount of the medicament or the pharmaceutical composition of the invention is in combination with Ursodeoxycholic acid (Ursodiol).

Therapeutic agents commonly used for treating inflammation related to transplant rejection (host versus graft disease) or Graft versus host disease are Calcineurins inhibitors (Cyclosporin, tacrolimus), mTOR inhibitors (Sirolimus, Everolimus), anti-proliferative agents (Azathioprine, Mycophenolic acid), monoclonal antibodies directed to CD25 (Dacluzimab, Basiliximab) or anti-thymocyte and anti-lymphocyte globulin preparations.

In a preferred embodiment of the present invention, the method for treating inflammation related to transplant rejection (host versus graft disease) or Graft versus host disease in a subject in need thereof comprises the administration to said subject of an effective amount of the medicament or the pharmaceutical composition of the invention in combination with Calcineurins inhibitors (Cyclosporin, tacrolimus), mTOR inhibitors (Sirolimus, Everolimus), anti-proliferative agents (Azathioprine, Methothrexate, Mycophenolic acid), monoclonal antibodies directed to CD25 (Dacluzimab, Basiliximab) or anti-thymocyte and anti-lymphocyte globulin preparations.

The present invention relates to the use of the pharmaceutical composition or medicament of the invention for treating inflammation related to transplant rejection (host versus graft disease) or Graft versus host disease in a subject in need thereof, wherein the administration to said subject of an effective amount of the medicament or the pharmaceutical composition of the invention is in combination with Calcineurins inhibitors (Cyclosporin, tacrolimus), mTOR inhibitors (Sirolimus, Everolimus), anti-proliferative agents (Azathioprine, Mycophenolic acid), monoclonal antibodies directed to CD25 (Dacluzimab, Basiliximab) or anti-thymocyte and anti-lymphocyte globulin preparations.

Therapeutic agents commonly used for treating asthma are short-acting, selective beta2-adrenoceptor agonists, such as salbutamol (albuterol USAN), levalbuterol, terbutaline and bitolterol and other adrenergic agonists such as inhaled epinephrine and ephedrine tablets, anticholinergic medications such as ipratropium bromide, inhaled glucocorticoids.

In a preferred embodiment of the present invention, the method for treating asthma in a subject in need thereof comprises the administration to said subject of an effective amount of the medicament or the pharmaceutical composition of the invention in combination with short-acting, selective beta2-adrenoceptor agonists such as salbutamol (albuterol USAN), levalbuterol, terbutaline and bitolterol and other adrenergic agonists such as inhaled epinephrine and ephedrine tablets, anticholinergic medications, such as ipratropium bromide, inhaled glucocorticoids.

The present invention relates to the use of the pharmaceutical composition or medicament of the invention for treating asthma in a subject in need thereof, wherein the administration to said subject of an effective amount of the medicament or the pharmaceutical composition of the invention is in combination with short-acting, selective beta2-adrenoceptor agonists such as salbutamol (albuterol USAN), levalbuterol, terbutaline and bitolterol and other adrenergic agonists, such as inhaled epinephrine and ephedrine tablets, anticholinergic medications such as ipratropium bromide, inhaled glucocorticoids.

In another embodiment of the present invention, the method for treating an inflammatory autoimmune condition in a subject in need thereof comprises the administration to said subject of an effective amount of the medicament or the pharmaceutical composition of the invention in combination with one or more medicament or pharmaceutical composition comprising at least one human Tr1 cell population directed against an antigen known to be involved in the inflammatory autoimmune condition.

The present invention relates to the use of the pharmaceutical composition or medicament of the invention, wherein the administration to said subject of an effective amount of the medicament or the pharmaceutical composition of the invention is in combination with one or more medicament or pharmaceutical composition comprising at least one human Tr1 cell population directed against an antigen known to be involved in the inflammatory autoimmune condition.

Examples of medicament or pharmaceutical composition that can be used for treating an arthritic condition, such as rheumatoid arthritis, ankylosing spondylitis, juvenile idiopathic arthritis and psoriatic arthritis, are the medicament or pharmaceutical composition comprising at least one Tr1 cell population or clone directed against a joint-associated antigen. Said joint-associated antigen is for example citrulline-substituted cyclic and linear filaggrin peptides, collagen type II peptides, human cartilage glycoprotein 39 (HCgp39) peptides, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

In a preferred embodiment, said human Tr1 cell population or clone is directed against a joint-associated antigen selected among type II collagen, HCgp39, fragments and variants thereof.

In a preferred embodiment of the invention, the method for treating an arthritic condition in a subject in need thereof comprises the administration to said subject of an effective amount of the medicament or the pharmaceutical composition of the invention in combination with an effective amount of a medicament or a pharmaceutical composition comprising at least one Tr1 cell population or clone directed against a joint-associated antigen, preferably against type II collagen, HCgp39, fragments, variants and mixtures thereof.

The present invention relates to the use of the pharmaceutical composition or medicament of the invention for treating an arthritic condition in a subject in need thereof, wherein the administration to said subject of an effective amount of the medicament or the pharmaceutical composition of the invention is in combination with an effective amount of a medicament or a pharmaceutical composition comprising at least one Tr1 cell population or clone directed against a joint-associated antigen, preferably against type II collagen, HCgp39, fragments, variants and mixtures thereof.

Examples of medicament or pharmaceutical composition that can be used for treating a multiple sclerosis condition are the medicament or pharmaceutical composition comprising at least one Tr1 cell population or clone directed against a multiple sclerosis-associated antigen. Said multiple sclerosis-associated antigen is for example myelin basic protein, myelin associated glycoprotein, myelin oligodendrocyte protein, proteolipid protein, oligodendrocyte myelin oligoprotein, myelin associated oligodendrocyte basic protein, oligodendrocyte specific protein, oligodendrocyte specific proteins, NOGO A, glycoprotein Po, peripheral myelin protein 22, 2'3'-cyclic nucleotide 3'-phosphodiesterase, and fragments, variants and mixtures thereof.

In a preferred embodiment, said human Tr1 cell population or clone is directed against a multiple sclerosis-associated antigen selected among myelin basic protein (MBP), proteolipid protein (PLP) and myelin oligodendrocyte protein (MOG) and fragments, variants and mixtures thereof.

In a preferred embodiment of the invention, the method for treating a multiple sclerosis condition in a subject in need thereof comprises the administration to said subject of an effective amount of the medicament or the pharmaceutical composition of the invention in combination with an effective amount of a medicament or a pharmaceutical composition comprising at least one Tr1 cell population or clone directed against a multiple sclerosis-associated antigen, preferably against myelin basic protein (MBP), proteolipid protein (PLP) and myelin oligodendrocyte protein (MOG) and fragments, variants and mixtures thereof.

The present invention relates to the use of the pharmaceutical composition or medicament of the invention for treating a multiple sclerosis condition in a subject in need thereof, wherein the administration to said subject of an effective amount of the medicament or the pharmaceutical composition of the invention is in combination with an effective amount of a medicament or a pharmaceutical composition comprising at least one Tr1 cell population or clone directed against a multiple sclerosis-associated antigen, preferably against myelin basic protein (MBP), proteolipid protein (PLP) and myelin oligodendrocyte protein (MOG) and fragments, variants and mixtures thereof.

Examples of medicament or pharmaceutical composition that can be used for treating an intestinal inflammatory condition, such as Crohn's disease and ulcerative colitis, are the medicament or pharmaceutical composition comprising at least one Tr1 cell population or clone directed against a food antigen from common human diet. Said food antigen from common human diet is for example bovine antigens, Ca-binding SIOO, alpha-lactalbumin, lactoglobulins, bovine serum albumin, immunoglobulin or caseins, atlantic salmon antigens, chicken antigens, ovalbumin, Ag22, conalbumin, lysozyme or chicken serum albumin, shrimp antigens, wheat antigens, celery antigens, carrot antigens, apple antigens, apple lipid transfer protein, apple profilin, pear antigens, isoflavone reductase, avocado antigens, apricot antigens, peach antigens, soybean antigens, peanuts, and fragments, variants and mixtures thereof.

In a preferred embodiment, said human Tr1 cell population or clone is directed against a food antigen from common human diet selected among ovalbumin, casein, beta-lactoglobulin, soya protein, gliadin, peanuts, and fragments, variants and mixtures thereof.

In a preferred embodiment of the invention, the method for treating an intestinal inflammatory condition in a subject in need thereof comprises the administration to said subject of an effective amount of the medicament or the pharmaceutical composition of the invention in combination with an effective amount of a medicament or a pharmaceutical composition comprising at least one Tr1 cell population or clone directed against a food antigen from common human diet, preferably against ovalbumin, casein, beta-lactoglobulin, soya protein, gliadin, peanuts, and fragments, variants and mixtures thereof.

The present invention relates to the use of the pharmaceutical composition or medicament of the invention for treating an intestinal inflammatory condition in a subject in need thereof, wherein the administration to said subject of an effective amount of the medicament or the pharmaceutical composition of the invention is in combination with an effective amount of a medicament or a pharmaceutical composition comprising at least one Tr1 cell population or clone directed against a food antigen from common human diet, preferably against ovalbumin, casein, beta-lactoglobulin, soya protein, gliadin, peanuts, and fragments, variants and mixtures thereof.

### EXAMPLES

In the following description, all experiments for which no detailed protocol is given are performed according to standard protocol.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXPERIMENTAL PROCEDURES

### TR1 CELL ISOLATION

Blood samples from healthy patients were collected and white blood cells were separated using gradient density centrifugation. Cells were then cultured in the presence of human HSP60 in order to induce the specific proliferation of Tr1 cells directed against this antigen. After 13 days of culture, cell populations were cloned by limiting dilution method.

Clones were then assessed for their specificity to human HSP60 and for characteristic Tr1 cytokine production profile.

### CYTOKINE ASSAYS

For the determination of antigen specificity, sandwich ELISAs were performed on 48 hours supernatants of Tr1 cell clones stimulated in the presence of antigen presenting cells (4.10⁵) and in the presence or absence of the specific antigen (HSP60). For the determination of the cytokine production profile, HSP60 Tr1 cell clones were stimulated with anti-CD3 + anti-CD28 monoclonal antibodies and the supernatants were harvested after 48 hours. ELISAs were performed using anti-IL-4 (11B11), anti-IL-10 (2A5), anti-IFN-γ (XGM1.2), biotin anti-IL-4 (24G2), anti-IL-10 (SXC1), anti-IFN-γ (R4-6A2) (Pharmingen Becton Dickinson).

### SUPPRESSION STUDIES

For suppression studies, autologous CD4 positive T lymphocytes were co-cultured with or without supernatants from activated HSP-60 specific Tr1 clones. Cultures were stimulated with anti-CD3 + anti-CD28 monoclonal antibodies and Interleukin-2. After 5 days total cell proliferation was assessed using the WST-1 proliferation kit from Roche.

### RESULTS

Figure 1 shows the IL-10 production of four distinct Tr1 cell populations specific for HSP60 in the presence or absence of the antigen. Results show that HSP60 stimulation induces an increase in the production of IL-10. These results demonstrate the specificity of the cell populations toward HSP60.

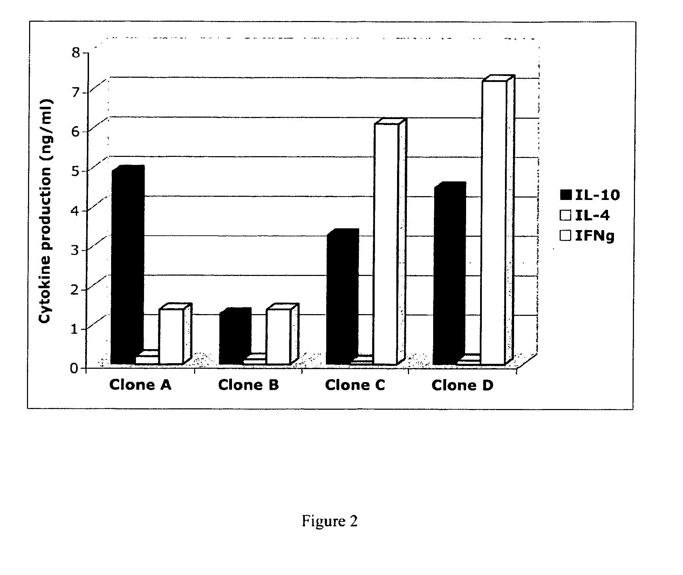

To further determine the cytokine secretion profile of these Tr1 cell populations specific for HSP60, cells were stimulated in the presence of anti-CD3+anti-CD28 monoclonal antibodies. ELISAs were performed on 48h supernatants to measure IL-4, IL-10 and IFNγ production. Figure 2 shows that the cytokine secretion profile observed for these HSP60 specific populations corresponds to a Tr1 cytokine secretion profile, i.e. high production of IL-10, production of IFNγ and no production of IL-4.

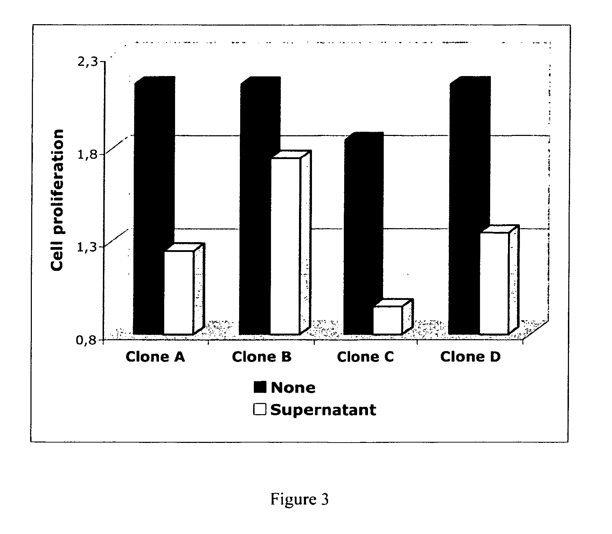

Suppressive activity of these HSP60 Tr1 populations was then assessed. Autologous CD4 positive T lymphocytes were co-cultured with or without supernatants from activated HSP-60 specific Tr1 clones. After 5 days of stimulation, cell proliferation was measured. Figure 3 shows the results for supernatants from the four Tr1 populations and confirms the suppressive activity of these Tr1 cells.

## Claims

1. Medicament comprising essentially at least one human Tr1 cell population directed against a human HSP.

2. Pharmaceutical composition comprising at least one human Tr1 cell population directed against a human HSP in combination with one or more pharmaceutically acceptable carrier.

3. A medicament according to claim 1 or a pharmaceutical composition according to claim **2**, wherein said human Tr1 cell population is a human Tr1 clone population.

4. A medicament or a pharmaceutical composition according to anyone of claim **1** to **3**, wherein said human Tr1 cell population is directed against HSP60, HSP70, HSP90 and mixtures thereof.

5. A medicament or a pharmaceutical composition according to anyone of claims **1 to 4**, for use in treating an inflammatory autoimmune condition.

6. The medicament or the pharmaceutical composition according to claim **5**, wherein said inflammatory autoimmune condition is an arthritis condition, preferably rheumatoid arthritis, psoriatic arthritis, ankylosing spondylitis and juvenile idiopathic arthritis.

7. The medicament or the pharmaceutical composition according to claim **5**, wherein said inflammatory autoimmune condition is a multiple sclerosis.

8. The medicament or the pharmaceutical composition according to claim **5**, wherein said inflammatory autoimmune condition is an intestinal inflammatory condition, preferably Crohn's disease and ulcerative colitis.

9. The medicament or the pharmaceutical composition according to claim **5**, wherein said inflammatory autoimmune condition is a vasculitis, preferably Wegener's disease and Atherosclerosis.

10. The medicament or the pharmaceutical composition according to claim **5**, wherein said inflammatory autoimmune condition is Asthma.

11. The medicament or the pharmaceutical composition according to claim **5**, wherein said inflammatory autoimmune condition is transplant rejection or Graft versus Host disease.

12. The medicament or the pharmaceutical composition according to claim **5**, wherein said inflammatory autoimmune condition is an inflammatory condition of the biliary duct, preferably Primary Biliary Cirrhosis and Primary sclerosing cholangitis.

13. The medicament or the pharmaceutical composition according to anyone of claims **5** to **12**, wherein the medicament or pharmaceutical composition to be administered to a subject in need thereof comprises human Tr1 cells autologous to the cells of said subject.

14. The medicament or the pharmaceutical composition according to anyone of claim **5** to **13**, wherein 10⁴/kg to 10⁹/kg Tr1 cells are administered to the subject in need thereof.

15. The medicament or the pharmaceutical composition according to anyone of claim **5** to **14**, wherein the administration to said subject of an effective amount of the medicament or the pharmaceutical composition is in combination with one or more therapeutic agent used for treating an inflammatory autoimmune condition.

16. The medicament or the pharmaceutical composition according to claim **15** for treating an arthritic condition, wherein the administration to said subject of an effective amount of the medicament or the pharmaceutical composition is in combination with one or more therapeutic agent selected in the group of corticoids, TNF blocking agents, anti-interleukins, anti-B lymphocytes, anti-costimulatory molecules, tolerogenic agents, anti-complement proteins, inhibitors of T cell signalling molecules, inhibitors of cell migration, leflunomide, sulfasalazine, hydroxychloroquine, azathioprine, methotrexate, cyclosporine, minocycline, D-penicillamine.

17. The medicament or the pharmaceutical composition according to claim **15** for treating a multiple sclerosis condition, wherein the administration to said subject of an effective amount of the medicament or the pharmaceutical composition is in combination with one or more therapeutic agent selected in the group of interferon-beta, glatiramer acetate, mitoxantrone, cyclophosphamide, methotrexate, azathioprine or natalizumab.

18. The medicament or the pharmaceutical composition according to claim **15** for treating an intestinal inflammatory condition, wherein the administration to said subject of an effective amount of the medicament or the pharmaceutical composition is in combination with one or more therapeutic agent selected in the group of TNF blocking agents, natalizumab, anti-ILl, anti-IL-6, anti- IL-12, anti-IL-17 and anti-IL-23; IL-1 receptor antagonist analogs (anakinra); 5 aminosalicyclic acid and analogs such as mesalazine, sulfazaline, olsalazine, balsalazide; corticoids such as prednisone, budesonide, hydrocortisone, prednisolone, methylprednisolone, betamethasone, bedomethasone, tixocortol.

19. The medicament or the pharmaceutical composition according to claim **15** for treating vasculitis, wherein the administration to said subject of an effective amount of the medicament or the pharmaceutical composition is in combination with one or more therapeutic agent selected in the group of statins, aspirin, blood coagulants, coumadin and corticoids, azathioprine, methothrexate, cyclophosphamide, anti-B lymphocytes antibodies, anti-TNF alpha antibodies and anti-thymocyte globulin.

20. The medicament or the pharmaceutical composition according to claim **15** for treating Asthma, wherein the administration to said subject of an effective amount of the medicament or the pharmaceutical composition is in combination with one or more therapeutic agent selected in the group of short-acting, selective beta2-adrenoceptor agonists, levalbuterol, terbutaline and bitolterol and other adrenergic agonists, anticholinergic medications and inhaled glucocorticoids.

21. The medicament or the pharmaceutical composition according to claim **15** for treating Graft versus host disease and transplant rejection, wherein the administration to said subject of an effective amount of the medicament or the pharmaceutical composition is in combination with one or more therapeutic agent selected in the group of Calcineurins inhibitors, mTOR inhibitors, anti-proliferative agents, monoclonal antibodies directed to CD25 or anti-thymocyte and antilymphocyte globulin preparations.

22. The medicament or the pharmaceutical composition according to claim **15** for treating autoimmune inflammation of the biliary duct or the liver such as Primary biliary cirrhosis and Primary sclerosing cholangitis, wherein the administration to said subject of an effective amount of the medicament or the pharmaceutical composition is in combination with one or more therapeutic agent selected in the group of Ursodeoxycholic acids.

23. The medicament or the pharmaceutical composition according to anyone of claim **5** to **14**, wherein the administration to said subject of an effective amount of the medicament or the pharmaceutical composition is in combination with one or more medicament or pharmaceutical composition comprising at least one Tr1 cell population or clone directed against an antigen known to be involved in the inflammatory autoimmune condition.

24. The medicament or the pharmaceutical composition according to claim **23** for treating an arthritic condition, wherein the administration to said subject of an effective amount of the medicament or the pharmaceutical composition is in combination with one or more medicament or pharmaceutical composition comprising at least one Tr1 cell population or clone directed against a joint-associated antigen, preferably against type II collagen, HCgp39, variants and mixtures thereof.

25. The medicament or the pharmaceutical composition according to claim **23** for treating a multiple sclerosis condition, wherein the administration to said subject of an effective amount of the medicament or the pharmaceutical composition is in combination with one or more medicament or pharmaceutical composition comprising at least one Tr1 cell population or clone directed against a multiple sclerosis-associated antigen, preferably against myelin basic protein, proteolipid protein, myelin oligodendrocyte protein, variants and mixtures thereof.

26. The medicament or the pharmaceutical composition according to claim **23** for treating an intestinal inflammatory condition, wherein the administration to said subject of an effective amount of the medicament or the pharmaceutical composition is in combination with one or more medicament or pharmaceutical composition comprising at least one Tr1 cell population or clone directed against a food antigen from human common diet, preferably against ovalbumin, casein, beta-lactoglobulin, soya protein, gliadin, peanuts, variants and mixtures thereof.

## Patentansprüche

1. Medikament, umfassend im Wesentlichen mindestens eine humane Tr1-Zellpopulation, die gegen ein humanes HSP gerichtet ist.

2. Pharmazeutische Zusammensetzung, umfassend mindestens eine humane Tr1-Zellpopulation, die gegen ein humanes HSP gerichtet ist, in Kombination mit einem oder mehreren pharmazeutisch annehmbaren Träger(n).

3. Medikament nach Anspruch 1 oder pharmazeutische Zusammensetzung nach Anspruch 2, wobei die humane Tr1-Zellpopulation eine humane Tr1-Klon-Population ist.

4. Medikament oder pharmazeutische Zusammensetzung nach einem beliebigen der Ansprüche 1 bis 3, wobei die humane Tr1-Zellpopulation gegen HSP60, HSP70, HSP90 und Gemische davon gerichtet ist.

5. Medikament oder pharmazeutische Zusammensetzung nach einem beliebigen der Ansprüche 1 bis 4 zur Verwendung beim Behandeln eines entzündlichen Autoimmunzustandes.

6. Medikament oder pharmazeutische Zusammensetzung nach Anspruch 5, wobei der entzündliche Autoimmunzustand ein Arthritis-Zustand ist, vorzugsweise rheumatoide Arthritis, psoriatische Arthritis, Spondylitis Ankylosans und juvenile idiopathische Arthritis.

7. Medikament oder pharmazeutische Zusammensetzung nach Anspruch 5, wobei der entzündliche Autoimmunzustand eine multiple Sklerose ist.

8. Medikament oder pharmazeutische Zusammensetzung nach Anspruch 5, wobei der entzündliche Autoimmunzustand ein intestinaler entzündlicher Zustand ist, vorzugsweise Morbus Crohn und Colitis ulcerosa.

9. Medikament oder pharmazeutische Zusammensetzung nach Anspruch 5, wobei der entzündliche Autoimmunzustand eine Vasculitis ist, vorzugsweise Wegener-Krankheit und Arteriosklerose.

10. Medikament oder pharmazeutische Zusammensetzung nach Anspruch 5, wobei der entzündliche Autoimmunzustand Asthma ist.

11. Medikament oder pharmazeutische Zusammensetzung nach Anspruch 5, wobei der entzündliche Autoimmunzustand Transplantatabstoßung oder Transplantat-Wirt-Reaktion ist.

12. Medikament oder pharmazeutische Zusammensetzung nach Anspruch 5, wobei der entzündliche Autoimmunzustand ein entzündlicher Zustand des Gallenganges ist, vorzugsweise primäre biliäre Zirrhose und primär sklerosierende Cholangitis.

13. Medikament oder pharmazeutische Zusammensetzung nach einem beliebigen der Ansprüche 5 bis 12, wobei das Medikament oder die pharmazeutische Zusammensetzung, das/ die an ein Subjekt, das diese(s) nötig hat, zu verabreichen ist, humane TR1-Zellen umfasst, die autolog zu den Zellen des Subjekts sind.

14. Medikament oder pharmazeutische Zusammensetzung nach einem beliebigen der Ansprüche 5 bis 13, wobei 10⁴/kg bis 10⁹/kg Tr1-Zellen an das Subjekt, das diese(s) nötig hat, verabreicht werden.

15. Medikament oder pharmazeutische Zusammensetzung nach einem beliebigen der Ansprüche 5 bis 14, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die verwendet wird/ werden zum Behandeln eines entzündlichen Autoimmunzustandes.

16. Medikament oder pharmazeutische Zusammensetzung nach Anspruch 15 zum Behandeln eines Arthritis-Zustandes, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von Kortikoiden, TNF-blockierenden Mitteln, Anti-Interleukinen, Anti-B-Lymphozyten, Anti-Costimulationsmolekülen, tolerogenen Mitteln, Anti-Komplement-Proteinen, Inhibitoren von T-Zell-Signalgebungsmolekülen, Inhibitoren von Zellmigration, Leflunomid, Sulfasalzin, Hydroxychloroquin, Azathioprin, Methothrexat, Cyclosporin, Minocyclin, D-Penicillamin.

17. Medikament oder pharmazeutische Zusammensetzung nach Anspruch 15 zum Behandeln eines Multiple-Sklerose-Zustandes, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von Interferon-beta, Glatirameracetat, Mitoxantron, Cyclophosphamid, Methothrexat, Azathioprin oder Natalizumab.

18. Medikament oder pharmazeutische Zusammensetzung nach Anspruch 15 zum Behandeln eines intestinalen entzündlichen Zustandes, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von TNF-Blockierungsmitteln, Natalizumab, Anti-IL1, Anti-IL-6, Anti-IL-12, Anti-IL-17 und Anti-IL-23; IL-1-Rezeptor-Antagonist-Analoga (Anakinra); 5-Aminosalicylsäure und Analoga, wie zum Beispiel Mesalazin, Sulfazalin, Olsalazin, Balsalazid; Corticoide wie z.B. Prednison, Budenosid, Hydrocortison, Prednisolon, Methylprednisolon, Betamethason, Bedomethason, Tixocortol.

19. Medikament oder pharmazeutische Zusammensetzung nach Anspruch 15 zum Behandeln von Vasculitis, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von Statinen, Aspirin, Blutkoagulantien, Coumadin und Corticoiden, Azathioprin, Methothrexat, Cyclophosphamid, Anti-B-Lymphozyten-Antikörpern, Anti-TNF-Alpha-Antikörpern und Anti-Thymocyt-Globulin.

20. Medikament oder pharmeztuische Zusammensetzung nach Anspruch 15 zum Behandeln von Asthma, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von kurzwirkenden selektiven Beta2-Adrenozeptor-Agonisten, Levalbuterol, Terbutalin und Bitolterol und anderen adrenergen Agonisten, anticholinergen Medikationen und inhalierten Glucocorticoiden.

21. Medikament oder pharmazeutische Zusammensetzung nach Anspruch 15, zum Behandeln von Transplantat-Wirt-Reaktion und Transplantat-Abstoßung, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von Calcineurine-Inhibitoren, mTOR-Inhibitoren, antiproliferativen Mitteln, monoklonalen Antikörpern, gerichtet gegen CD25, oder anti-Thymozyt- und Anti-Lymphozyt-Globulin-Zubereitungen.

22. Medikament oder pharmazeutische Zusammensetzung nach Anspruch 15 zum Behandeln von Autoimmunentzündung des Gallenganges oder der Leber, wie z.B. primärer biliärer Zirrhose und primär sklerosierender Cholangitis, wobei die Verabreichung einer wirksamen Menge des Medikaments oder der pharmazeutischen Zusammensetzung an das Subjekt in Kombination mit einem oder mehreren therapeutischen Mittel(n) erfolgt, das/ die ausgewählt ist/ sind in der Gruppe von Ursodesoxycholsäuren.

23. Medikament oder pharmazeutische Zusammensetzung gemäß einem beliebigen der Ansprüche 5 bis 14, wobei die Verabreichung einer wirksamen Menge des Medikaments oder der pharmazeutischen Zusammensetzung an das Subjekt in Kombination mit einem/ einer oder mehreren Medikament(en) oder pharmazeutischen Zusammensetzung(en) erfolgt, umfassend mindestens eine(n) Tr1-Zellpopulation oder -Klon, gerichtet gegen ein Antigen, von dem bekannt ist, dass es an dem entzündlichen Autoimmunzustand beteiligt ist.

24. Medikament oder pharmazeutische Zusammensetzung nach Anspruch 23 zum Behandeln eines Arthritis-Zustandes, wobei die Verabreichung einer wirksamen Menge des Medikaments oder der pharmazeutischen Zusammensetzung an das Subjekt in Kombination mit einem/ einer oder mehreren Medikament(en) oder pharmazeutischen Zusammensetzung(en) erfolgt, umfassend mindestens eine(n) Tr1- Zellpopulation oder -Klon, gerichtet gegen einen gelenkassoziiertes Antigen, vorzugsweise gegen Typ-II-Kollagen, HCgp39, Varianten und Gemische davon.

25. Medikament oder pharmazeutische Zusammensetzung nach Anspruch 23 zum Behandeln eines Multiple-Sklerose-Zustandes, wobei die Verabreichung einer wirksamen Menge des Medikaments oder der pharmazeutischen Zusammensetzung an das Subjekt in Kombination mit einem/ einer oder mehreren Medikament(en) oder pharmazeutischen Zusammensetzung(en) erfolgt, umfassend mindestens eine(n) Tr1-Zellpopulation oder -Klon, gerichtet gegen einen Multiple-Sklerose-assoziiertes Antigen, vorzugsweise gegen Myelin-Basisches Protein, Proteolipid-Protein, Myelin-Oligodendrozyt-Protein, Varianten und Gemische davon.

26. Medikament oder pharmazeutische Zusammensetzung nach Anspruch 23 zum Behandeln eines intestinalen entzündlichen Zustandes, wobei die Verabreichung einer wirksamen Menge des Medikaments oder der pharmazeutischen Zusammensetzung an das Subjekt in Kombination mit einem/ einer oder mehreren Medikament(en) oder pharmazeutischen Zusammensetzung(en) erfolgt, umfassend mindestens eine(n) Tr1-Zellpopulation oder -Klon, gerichtet gegen ein Nahrungsmittelantigen von einer gewöhnlichen humanen Ernährung, vorzugsweise gegen Ovalbumin, Kasein, Beta-Lactoglobulin, Sojaprotein, Gliadin, Erdnüsse, Varianten und Gemische davon.

## Revendications

1. Médicament comprenant essentiellement au moins une population de cellules Tr1 humaine dirigée contre une HSP humaine.

2. Composition pharmaceutique comprenant au moins une population de cellules Tr1 humaine dirigée contre une HSP humaine en combinaison avec un ou plusieurs véhicules pharmaceutiquement acceptables.

3. Un médicament selon la revendication **1** ou une composition pharmaceutique selon la revendication **2**, dans lequel ladite population de cellules Tr1 humaine est une population de cellules Tr1 humaine clonale.

4. Un médicament ou une composition pharmaceutique selon l'une quelconque des revendications **1** à **3**, dans lequel ladite population de cellules Tr1 humaine est dirigée contre HSP60, HSP70, HSP90 et leurs mélanges.

5. Un médicament ou une composition pharmaceutique selon l'une quelconque des revendications **1** à **4**, pour une utilisation dans le traitement d'une condition autoimmune inflammatoire.

6. Le médicament ou la composition pharmaceutique selon la revendication **5**, dans lequel ladite condition autoimmune inflammatoire est une condition arthritique, préférentiellement une polyarthrite rhumatoïde, une arthrite psoriasique, une spondylarthrite ankylosante ou une arthrite juvénile idiopathique.

7. Le médicament ou la composition pharmaceutique selon la revendication **5**, dans lequel ladite condition autoimmune inflammatoire est une sclérose en plaques.

8. Le médicament ou la composition pharmaceutique selon la revendication **5**, dans lequel ladite condition autoimmune inflammatoire est une condition inflammatoire intestinale, préférentiellement une maladie de Crohn ou une colite ulcéreuse.

9. Le médicament ou la composition pharmaceutique selon la revendication **5**, dans lequel ladite condition autoimmune inflammatoire est une vascularite, préférentiellement une maladie de Wegener ou une athérosclérose.

10. Le médicament ou la composition pharmaceutique selon la revendication **5**, dans lequel ladite condition autoimmune inflammatoire est un asthme.

11. Le médicament ou la composition pharmaceutique selon la revendication **5**, dans lequel ladite condition autoimmune inflammatoire est un rejet de greffe ou une maladie du greffon contre l'hôte.

12. Le médicament ou la composition pharmaceutique selon la revendication **5**, dans lequel ladite condition autoimmune inflammatoire est une condition inflammatoire de la voie biliaire, de préférence une cirrhose biliaire primitive ou une cholangite sclérosante primitive.

13. Le médicament ou la composition pharmaceutique selon l'une quelconque des revendications **5** à **12**, dans lequel le médicament ou la composition pharmaceutique à administrer à un sujet en ayant besoin comprend des cellules Tr1 humaines autologues aux cellules dudit sujet.

14. Le médicament ou la composition pharmaceutique selon l'une quelconque des revendications **5** à **12**, dans lequel 10⁴/kg à 10⁹/kg de cellules Tr1 sont administrées au sujet en ayant besoin.

15. Le médicament ou la composition pharmaceutique selon l'une quelconque des revendications **5** à **14**, dans lequel l'administration audit sujet d'une quantité efficace du médicament ou de la composition pharmaceutique est en combinaison avec un ou plusieurs agents thérapeutiques utilisés pour traiter une condition autoimmune inflammatoire.

16. Le médicament ou la composition pharmaceutique selon la revendication **15** pour traiter une condition arthritique, dans lequel l'administration audit sujet d'une quantité efficace du médicament ou de la composition pharmaceutique est en combinaison avec un ou plusieurs agents thérapeutiques sélectionnés dans le groupe des corticoïdes, agents bloqueurs du TNF, anti-interleukines, anti-lymphocytes B, molécules anti-costimulation, agents tolérogéniques, protéines anti-compléments, inhibiteurs de molécules de la signalisation des cellules T, inhibiteurs de la migration cellulaire, du léflunomide, sulfasalazine, hydroxychloroquine, azathioprine, méthotrexate, ciclosporine, minocycline, D-pénicillamine.

17. Le médicament ou la composition pharmaceutique selon la revendication **15** pour traiter une condition de sclérose en plaques, dans lequel l'administration audit sujet d'une quantité efficace du médicament ou de la composition pharmaceutique est en combinaison avec un ou plusieurs agents thérapeutiques sélectionnés dans le groupe de l'interféron-beta, acétate de glatiramère, mitoxantrone, cyclophosphamide, méthotrexate, azathioprine ou natalizumab.

18. Le médicament ou la composition pharmaceutique selon la revendication **15** pour traiter une condition inflammatoire intestinale, dans lequel l'administration audit sujet d'une quantité efficace du médicament ou de la composition pharmaceutique est en combinaison avec un ou plusieurs agents thérapeutiques sélectionnés dans le groupe des agents bloqueurs du TNF, natalizumab, anti-IL-1, anti-IL-6, anti-IL-12, anti-IL-17 et anti-IL-23 ; analogues d'antagonistes du récepteur à l'IL-1 (anakinra), acide 5-aminosalicylique et ses analogues comme la mésalazine, sulfazaline, olsalazine, balsalazide ; corticoïdes comme la prednisone, budésonide, hydrocortisone, prednisolone, méthylprednisolone, bétaméthasone, bédométhasone, tixocortol.

19. Le médicament ou la composition pharmaceutique selon la revendication **15** pour traiter une vascularite, dans lequel l'administration audit sujet d'une quantité efficace du médicament ou de la composition pharmaceutique est en combinaison avec un ou plusieurs agents thérapeutiques sélectionnés dans le groupe des statines, aspirine, coagulants sanguins, Coumadin et corticoïdes, azathioprine, méthotrexate, cyclophosphamide, anticorps anti-lymphocytes B, anticorps anti-TNF alpha et globuline antithymocyte.

20. Le médicament ou la composition pharmaceutique selon la revendication **15** pour traiter un asthme, dans lequel l'administration audit sujet d'une quantité efficace du médicament ou de la composition pharmaceutique est en combinaison avec un ou plusieurs agents thérapeutiques sélectionnés dans le groupe des agonistes sélectifs béta 2-adrénergiques à courte durée d'action, lévalbutérol, terbutaline et bitoltérol et autres agonistes adrénergiques, médicaments anticholinergiques et glucocorticoïdes inhalés.

21. Le médicament ou la composition pharmaceutique selon la revendication **15** pour traiter une maladie du greffon contre l'hôte ou un rejet de greffe, dans lequel l'administration audit sujet d'une quantité efficace du médicament ou de la composition pharmaceutique est en combinaison avec un ou plusieurs agents thérapeutiques sélectionnés dans le groupe des inhibiteurs des calcineurines, inhibiteurs de mTOR, agents anti-prolifératifs, anticorps monoclonaux dirigés contre CD25 ou anti-thymocytes et préparations de globulines anti-lymphocytes.

22. Le médicament ou la composition pharmaceutique selon la revendication **15** pour traiter une inflammation autoimmune de la voie biliaire ou du foie, comme une cirrhose biliaire primitive et une cholangite sclérosante primitive, dans lequel l'administration audit sujet d'une quantité efficace du médicament ou de la composition pharmaceutique est en combinaison avec un ou plusieurs agents thérapeutiques sélectionnés dans le groupe des acides ursodésoxycholiques.

23. Le médicament ou la composition pharmaceutique selon l'une quelconque des revendications **5** à **14**, dans lequel l'administration audit sujet d'une quantité efficace du médicament ou de la composition pharmaceutique est en combinaison avec un ou plusieurs médicaments ou compositions pharmaceutiques comprenant au moins une population ou un clone de cellules Tr1 dirigées contre un antigène connu pour être impliqué dans une condition autoimmune inflammatoire.

24. Le médicament ou la composition pharmaceutique selon la revendication **23** pour traiter une condition arthritique, dans lequel l'administration audit sujet d'une quantité efficace du médicament ou de la composition pharmaceutique est en combinaison avec un ou plusieurs médicaments ou compositions pharmaceutiques comprenant au moins une population ou un clone de cellules Tr1 dirigées contre un antigène associé aux articulations, préférentiellement contre le collagène de type II, HCgp39, leurs variantes et leurs mélanges.

25. Le médicament ou la composition pharmaceutique selon la revendication **23** pour traiter une condition de sclérose en plaques, dans lequel l'administration audit sujet d'une quantité efficace du médicament ou de la composition pharmaceutique est en combinaison avec un ou plusieurs médicaments ou compositions pharmaceutiques comprenant au moins une population ou un clone de cellules Tr1 dirigées contre un antigène associé à une sclérose en plaques, préférentiellement contre une protéine basique myéline, une protéine protéolipidique, une protéine oligodendrocyte myéline, leurs variantes et leurs mélanges.

26. Le médicament ou la composition pharmaceutique selon la revendication **23** pour traiter une condition inflammatoire intestinale, dans lequel l'administration audit sujet d'une quantité efficace du médicament ou de la composition pharmaceutique est en combinaison avec un ou plusieurs médicaments ou compositions pharmaceutiques comprenant au moins une population ou un clone de cellules Tr1 dirigées contre un antigène alimentaire de l'alimentation humaine commune, préférentiellement contre l'ovalbumine, la caséine, la béta-lactoglobuline, les protéines de soja, les gliadines, les cacahuètes, leurs variantes et leurs mélanges.
